# EUROPEAN PATENT APPLICATION

(11) **EP 1 516 642 A1**
(43) Date of publication of application: **23.03.2005**
(21) Application number: 04445092.2
(22) Date of filing: 10.09.2004
(51) Int. Cl.: A61M 16/00, F16L 37/08

(54) **Gas saver for breathing gas**

(30) Priority: 10.09.2003 SE 0302406
(71) Applicant: GCE GAS CONTROL EQUIPMENT AB, S-211 20 Malmö (SE)
(72) Inventor: Kihlberg, Yngve, 277 32 Kivik (SE)
(74) Representative: Rostovanyi, Peter

(57) **Abstract**

A gas-saving inhalation-controlled device which has a breathing gas volume for inhalation originating from a pressurised gas cylinder with pressure-reducing means (20, 21), said breathing gas volume being supplied for inhalation through a solenoid valve (4) which is controlled by electronics. The breathing gas volume is defined essentially by the boundary wall of a chamber (7) in a casing (1) which holds the solenoid valve (4) and the control electronics and which is connectible to the outlet of the pressure-reducing means directly by means of a short connecting piece (8) arranged on the casing, or by means of a short tube, where the short tube does not have a significant volume in relation to the breathing volume.

## Description

The present invention relates to a breathing-gas-saving device according to claim 1.

Breathing gas is frequently supplied to a user in need from a gas cylinder or a gas generating apparatus which is connected to the user by a tube. The gas cylinder has an opening/shut-off valve, and a pressure reducer ensures that the pressure of the gas cylinder is reduced to a level suitable for inhalation. The gas is usually oxygen but other gases or gas mixtures can be used.

DE-296 22 321.2 discloses such a device for dosed supply of gas to a user, said device saving gas by supplying gas during inhalation only, and letting the user's need for inhalation control the supplied amount of gas. To this end, the device comprises a sensor and associated electronics, the sensor detecting the negative pressure in inhalation and controlling a solenoid valve for opening, and the electronics ensuring that the duration of the opening state of the valve is controlled by the breathing frequency, the electronics possibly also determining the amount of gas supplied. The gas cylinder and the solenoid valve are interconnected by means of a long tube. The gas cylinder stores breathing gas at high pressure.

A drawback of such a device resides in the large volume of gas that is accumulated in the tube, which large volume is a gas buffer. A further drawback is that different outlet pressures from the gas cylinder (from the pressure-reducer of the gas cylinder) produce different pressure losses in the tube during inhalation, which in turn results in the need of exchanging the control electronics or reprogramming thereof, so that the patient receives the correct breathing volume at correct time intervals during inhalation.

The object of the invention is to eliminate or at least reduce the above drawbacks in a device of the type described above with dosed supply of breathing gas.

A further object is to provide a practically manageable gas-saving device for breathing gas or treatment gas.

The object is achieved by a gas-saving device having the features as stated in claim 1, advantageous embodiments having the features stated in the dependent claims.

In other words, there is provided according to the invention a gas-saving, inhalation-controlled device with an integrated gas volume with a small pressure drop.

An embodiment of the invention will now be described in more detail with reference to the accompanying illustrations, in which
Fig. 1 is a view from the connection side of a device according to the invention;
Fig. 2 is a view from the opposite side;
Fig. 3 is an exploded view of the device;
Fig. 4 is a front view of the schematically illustrated outlet of a prior-art pressure regulator for breathing gas bottles;
Fig. 5 is an arrangement of the device; and
Fig. 6 is a cross-sectional view of a pressure reducer in the arrangement according to Fig. 5.

The device according to the invention comprises a casing 1, which consists of two trough-shaped parts 1a, 1b and in which are mounted a sensor 2, electronics (integrated printed circuit board) 3, a solenoid valve 4 with an outlet 4a and power supply in the form of a rechargeable battery 5. The casing part 1b and a partition wall 6 of the casing define between them a gas chamber 7 which communicates with the outside of the casing 1 by means of a quick connecting piece 8. This is arranged for direct quick connection to the low pressure outlet of a pressure reducer or regulator of a gas cylinder (not shown), mounted on the gas cylinder. The solenoid valve 4 is mounted on the partition wall 6, on its side facing away from the chamber 7, so that its inlet (not shown) communicates with the chamber 7. The outlet 4a of the solenoid valve 4 in the form of a connecting piece is arranged to be connected to one end of a tube, whose other user end has a nose halter to be connected to the user's nose. The solenoid valve 4 is arranged to open, i.e. make the chamber 7 communicate with the patient, through said outlet 4a and said tube, during the patient's inhalation, in the same way as in the above-described prior-art device. The electronics 3 and the sensor 2 are mounted in the space between the partition wall 6 and the casing part 1a, and the electronics can be designed in the same way as in the above-described prior-art device.

The quick connecting piece 8 has a thread 8a on its rear part which opens into the gas chamber 7, and is fastened to the casing part 1b by means of a nut (not shown). On the outside of the casing 1 there is a disengaging unit 10 which is adapted to disengage the quick connecting piece 8 and thus the device with its casing 1 from the low pressure outlet of the pressure reducer of the gas cylinder.

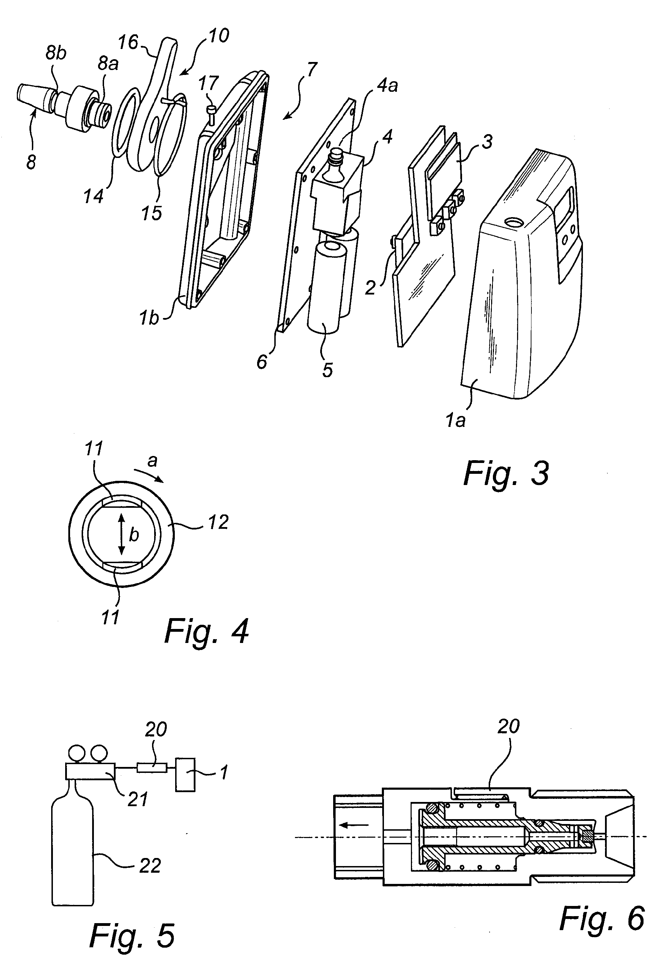

The pressure regulator in the embodiment shown is of a type that is mounted on a valve unit marketed by GCE Gas Control Equipment AB and designated Combilite®, which has a shut-off valve, a manometer, a gas supply port and is screwable onto a gas cylinder. The pressure regulator is schematically shown in a front view in Fig. 4. Such a pressure regulator has in its low pressure outlet spring-biased tongues 11 which project radially in the outlet duct and are movable apart, against spring action, by turning a ring 12 at said outlet, but also radially outwards from the position shown, as indicated by arrows A and B.

The quick connecting piece 8 has a groove 8b for engagement with the tongues 11 by snap action. In this engaging position, the device (i.e. the entire casing 1) is held on the gas cylinder, and to ensure immobility of the device on the gas cylinder there may be supplementary holding means, here in the form of grooves 13 on the casing 1 for engagement with flanges on a normal valve-protecting cap on the gas cylinder.

The disengaging unit 10 comprises a friction ring 14 of, for example, rubber material, and a torsion spring 15 which is fixed to a disengaging arm 16 at one end and to the casing 1 at the other end and is arranged so that turning of the disengaging arm 16 for disengagement occurs against the action of the restoring spring 15, which in turn results in the arm 16, after disengagement, being automatically returned to its unloaded position. The friction ring 14 is arranged in a groove in the arm 16 and adapted to frictionally engage the outer surface of the turnable ring 12 of the pressure reducer.

The casing parts are releasably assembled by means of screws and, of course, suitable seals.

The gas chamber 7 has a volume suited for correct inhalation-controlled gas supply, i.e. a volume which allows a correct, suitable predetermined volume of gas to be quickly pressed into the lungs and which allows quick accumulation of the correct gas volume originating from the gas cylinder, at the correct pressure in the gas chamber 7. It has been found that, by being close to the gas cylinder and the solenoid valve 4, a gas chamber 7 with a volume of 35 cm³ can replace a conventionally used 4.5 m tube with a volume of 50 cm³, thereby saving space and eliminating the risk of entangling the tube.

In some situations, it may still be preferred to connect the gas cylinder to the solenoid valve 4 by means of a tube. In the described device, the quick connecting piece 8 and the disengaging unit are dismounted from the casing 1, and the hole left in the casing part 1b for the quick coupling 8 (intended for the gas) is stopped up by a plug, and the gas chamber 7 is connected to the pressure reducer by means of a tube which thanks to the existence and use of the gas chamber 7 in the above-described casing 1 can be short, so that the device - with the quick connecting piece and the disengaging unit removed - can be carried in the user's pocket or be attached to a pocket or a belt by means of a clip fixed to the casing 1. To this end, the casing 1 can be provided with a tube-connectible inlet 17 which communicates with the gas chamber 7 and is stopped up when the casing 1 is connected directly, as described above, to the outlet of the pressure reducer. If the pressure reducer is of the type described above with an outlet to be connected to a quick coupling of the type as described above, the inlet 17 should of course be connected to the pressure reducer by means of a tube whose other end is connected to the quick coupling (of the type 8).

It will be appreciated that the device according to the invention is adjustable to different types of pressure regulators, which are available on the market with different permanent (set at the factory) output pressures or variable output pressures. They can be integrated units, like in the above-described Combilite® - with a shut-off valve (for the gas cylinder), a pressure regulator and a cylinder filling valve integrated in a body or separately connectible to a shut-off valve. Variants allowing the flow to be regulated are also available on the market. The device according to the invention can, of course, be modified for connecting its chamber 7 to all these different types of valves/regulators.

An arrangement is illustrated in Fig. 5, which serves to allow use of the inventive device together with different pressure regulators with different output pressures, where the output pressure is higher than a predetermined pressure value, at which the device according to the invention operates. The arrangement comprises an additional pressure reducer or adaptor 20 (Fig. 6) which is adapted to supply the constant predetermined pressure value to the chamber 7, independently of the input pressure from the ordinary pressure regulator 21. Such pressure reducers (of the type described above as additional pressure reducer) are also known to those skilled in the art. The gas cylinder is designated 22.

It will be appreciated that the disengaging unit 10 need not be designed as shown and described above, but also other types of disengaging arrangements are possible, according to the design of the outlet of the pressure regulator/reducer, not all of them having precisely the above-described tongue arrangement for quick coupling (intended for quick coupling to a tube).

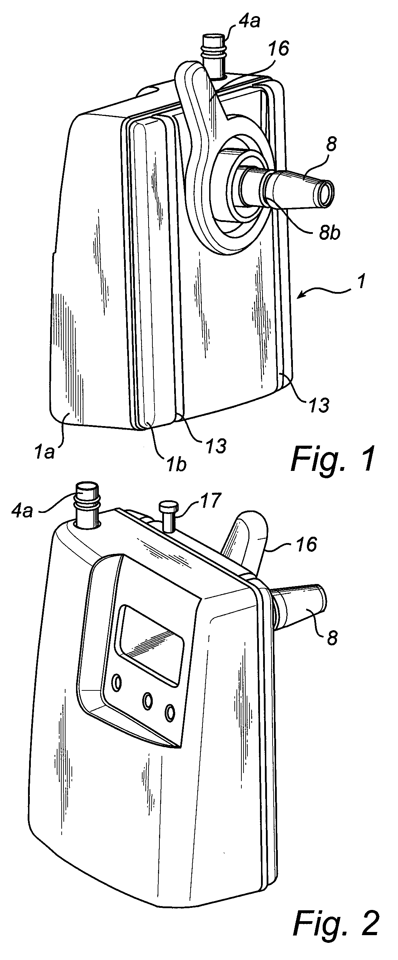

Fig. 2 shows a display and set buttons.

## Claims

1. A gas-saving inhalation-controlled device which has a breathing gas volume for inhalation originating from a pressurised gas cylinder or a gas generating apparatus with pressure-reducing means (20, 21), said breathing gas volume being supplied for inhalation through a solenoid valve (4) controlled by electronics, **characterised in that** the breathing gas volume is defined essentially by the boundary wall of a chamber (7) in a casing (1) which holds the solenoid valve (4) and the control electronics and which is connectible to the outlet of the pressure-reducing means directly by means of a short connecting piece (8) arranged on the casing, or by means of a short tube, where the short tube does not have a significant volume related to the breathing volume.

2. A device as claimed in claim 1, **characterised in that** it comprises a pressure reducer (20) adapted to supply a predetermined constant breathing gas pressure to the chamber (7), said pressure reducer being arranged to be connected to the pressure-reducing means of the gas cylinder.

3. A device as claimed in claim 1, **characterised in that** the connecting piece (8) is a quick coupling (8).

4. A device as claimed in claim 3, **characterised in that** the quick coupling (8) is a snap-in fastener for cooperation with spring-biased tongues (11) of the outlet of the pressure reducer.

5. A device as claimed in claim 3 or 4, **characterised in that** a spring-biased disengaging unit (11) for the quick coupling (8) is mounted on the casing (1), when the quick coupling engages said outlet.
